(19) Europäisches Patentamt
European Patent Office
Office européen des brevets

(11) Veröffentlichungsnummer : **0 103 193**
**B1**

(12) # EUROPÄISCHE PATENTSCHRIFT

(45) Veröffentlichungstag der Patentschrift : **11.12.85**

(21) Anmeldenummer : **83108044.5**

(22) Anmeldetag : **13.08.83**

(51) Int. Cl.⁴ : **C 07 D211/58**, C 07 D211/46, C 07 D211/22, C 07 D211/26, C 07 D211/28, C 08 K 5/34

(54) **Quadratsäureamidderivate, ihre Verwendung als Stabilisierungsmittel sowie die mit diesen behandelten Kunststoffe.**

(30) Priorität : **14.09.82 DE 3233954**

(43) Veröffentlichungstag der Anmeldung : **21.03.84 Patentblatt 84/12**

(45) Bekanntmachung des Hinweises auf die Patenterteilung : **11.12.85 Patentblatt 85/50**

(84) Benannte Vertragsstaaten :
**AT BE CH DE FR GB IT LI NL SE**

(56) Entgegenhaltungen :
**DE-A- 2 638 855**
**DE-A- 3 003 843**
**DE-A- 3 111 739**
**DE-A- 3 201 415**

(73) Patentinhaber : **HÜLS AKTIENGESELLSCHAFT**
**Patentabteilung / PB 15 - Postfach 13 20**
**D-4370 Marl 1 (DE)**

(72) Erfinder : **Rombusch, Konrad, Dr.**
**Flämingstrasse 5**
**D-4370 Marl (DE)**
Erfinder : **Maahs, Günther, Dr.**
**Oderbruchstrasse 19**
**D-4370 Marl (DE)**
Erfinder : **Schäfer, Wolfgang, Dr.**
**Wiesenstrasse 71**
**D-4370 Marl (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 103 193 B1

## Beschreibung

Es ist bekannt, daß Kunststoffe unter der Einwirkung von Licht und Wärme sowie unter dem Einfluß der natürlichen Bewitterung dazu neigen zu vergilben, spröde zu werden und sich schließlich ganz zu zersetzen. Der dadurch verursachte Schaden kann erhebliche Ausmaße annehmen. Schon seit längerer Zeit werden daher Kunststoffen, die besonders starken Umwelteinflüssen ausgesetzt sind, eine Reihe von Stabilisatoren zugesetzt.

Es handelt sich dabei um chemisch sehr unterschiedliche Verbindungen, die nach verschiedenen, im Detail vielfach unbekannten Mechanismen eine Stabilisierung der Kunststoffe gegenüber den natürlichen Umwelteinflüssen bewirken. So sind aus den DE-OSS 26 06 026 und 26 34 957 1-Oxa-4,8-diaza-spiro-(4,5)-decane bekannt, die den Polymeren neben weiteren üblichen Stabilisatoren zugesetzt werden. Die auf dem Markt vertretenen Typen unter der Bezeichnung TINUVIN® 770 (siehe Vergleichsbeispiel A) bleiben in ihrer lichtstabilisierenden Wirkung hinter den Erwartungen zurück.

Die lichtstabilisierende Wirkung von Tetramethylpiperidin und seinen Derivaten ist darauf zurückzuführen, Radikalzwischenprodukte des Photooxidationsprozesses vollständig abzufangen [vgl. J. Polym. Sci. Polym. Chem. Ed. *19*, 807 (1981)].

Auch bestimmte Quadratsäure-1,3-bisamide haben sich aufgrund ihrer Absorber- und Quencherwirkung in Witterungstests als wirkungsvolle Stabilisatoren erwiesen (vgl. z.B. DE-OS 26 38 855).

Alle bekannten Stabilisatoren bzw. Stabilisatorkombinationen sind jedoch mit Mängeln behaftet, die sich besonders bei dünnen Kunststoffschichten bemerkbar machen :

1. Die bekannten Stabilisatoren sind zu flüchtig.
2. Sie sind leicht extrahierbar.
3. Sie weisen eine erhebliche Migrationsneigung auf.

Es stellte sich daher die Aufgabe, Stabilisatoren zu entwickeln, die diese Nachteile nicht aufweisen.

Es wurde jetzt überraschend gefunden, daß die im Anspruch aufgeführten, durch Tetramethylpiperidin substituierten Quadratsäure-1,3-bisamide bzw. deren Salze eine dauerhafte und verbesserte Stabilisatorwirkung entfalten.

Gegenstand der Erfindung ist demnach auch die Verwendung dieser Verbindungen als Stabilisatoren. Ferner wird für die mit dem Stabilisator behandelten Kunststoffe Schutz begehrt.

In der DE-OS 26 38 855 wird bereits ein Quadratsäureamid aufgeführt, das durch einen Tetramethylpiperidinring substituiert ist. Das dort beschriebene Quadratsäure-1,3-bis-2',2',6',6'-tetramethylpiperidon-(4')-id unterscheidet sich nicht nur strukturell von den hier beschriebenen Derivate ; es sind auch keine Synthesen dieser Verbindung bekannt.

Ausgangsverbindungen für die Synthese sind Tetramethylpiperidinderivate der Formel

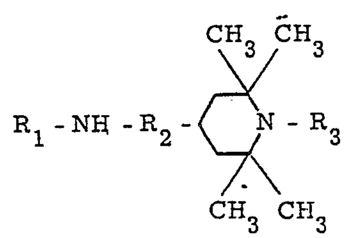

mit $R_2 = —(CH_2)_m—(X)_n—$, X = O oder NH, m = 0 bis 6 und n = 0 oder 1.

$R_1$ kann Wasserstoff oder ein gegebenenfalls durch eine Hydroxylgruppe substituierter geradkettiger oder verzweigter Alkylrest mit 1 bis 12 C-Atomen sein, beispielsweise ein Hydroxyethyl-, Propyl-, Isopropyl-, n- oder iso-Butyl-, Hexyl-, Decyl- oder Dodecylrest.

Wie aus der Formel ersichtlich, kann der Tetramethylpiperidinring entweder direkt oder über den $R_2$-Rest mit der $R_1$-substituierten Aminogruppe verbunden sein.

$R_2$ ist ein Alkylenrest mit bis zu 6 C-Atomen, der gegebenenfalls durch eine Ethergruppe oder eine Aminogruppe verlängert sein kann. Beispielsweise ist $R_2$ der Oxytrimethylenrest.

$R_3$ kann schließlich Wasserstoff, ein Methyl-, ein Hydroxyethyl- oder ein Carboxymethyl- oder -ethylrest sein.

Die genannten Verbindungen können auf vielen, an sich bekannten Synthesewegen hergestellt werden. Im Falle von m und n = 0 ist beispielsweise das nach der DE-OS 30 03 843 erhältliche Triacetondiamin ein wichtiges Zwischenprodukt.

Die Umsetzung der Tetramethylpiperidinderivate mit Quadratsäure bei erhöhter Temperatur erfolgt analog zur DE-OS 26 38 855. Arbeitet man in Gegenwart eines Lösemittels, so sollte man das Tetramethylpiperidinderivat in mindestens doppelt molarer Menge einsetzen. Verwendet man das Tetramethylpiperidinderivat als Lösemittel, so kann ein bis zu 30facher Überschuß eingesetzt werden. Die Reaktionstemperatur liegt zwischen 100 und 250 °C.

Soweit erwünscht, können anschließend die Salze der Quadratsäurebisamide hergestellt werden. Dazu wird eine Mischung aus dem Quadratsäurebisamid und maximal zwei Äquivalenten Säure bei einer

Temperatur von höchstens 200 °C solange erhitzt, bis das IR-Spektrum das Produkt als Salz ausweist.

Als Säurekomponente eignen sich ein- und mehrbasige organische Säuren mit bis zu 20 C-Atomen, beispielsweise Essigsäure, Laurinsäure, Stearinsäure, Ölsäure, Bernsteinsäure, Adipinsäure, Dodecan-1,12-disäure, Phthalsäure, Citronensäure sowie Phosphorsäure, Schwefelsäure, Phenylphosphonsäure und Dodecansulfonsäure.

Es wird in den meisten Fällen ausreichend sein, als Stabilisatoren die Quadratsäure-1,3-bisamide einzusetzten. Dort, wo es auf extrem niedrige Migrationsneigung ankommt, empfiehlt es sich, die Salze einzusetzen.

Beispiele für die beanspruchten Verbindungen sind :

1,3-Bis-[N-n-propyl-N-(2,2,6,6-tetramethyl-piperidyl-(4))-amino]-cyclobuten-diylium-2,4-diolat,
1,3-Bis-[N-n-octyl-N-(2,2,6,6-tetramethyl-piperidyl-(4))-amino]-cyclobuten-diylium-2,4-diolat,
1,3-Bis-[N-n-octadecyl-N-(2,2,6,6-tetramethyl-piperidyl-(4))-amino]-cyclobuten-diylium-2,4-diolat,
1,3-Bis-[2,2,6,6-tetramethyl-piperidyl-(4)-aminopropylamino]-cyclobuten-diylium-2,4-diolat,
1,3-Bis-[1,2,2,6,6-pentamethyl-piperidyl-(4)-amino]-cyclobutendiylium-2,4-diolat,
1,3-Bis-[(1-hydroxy-ethyl-2,2,6,6-tetramethyl-piperidyl-(4))-amino]-cyclobuten-diylium-2,4-diolat.

Bevorzugt sind :

1,3-Bis-[2,2,6,6-tetramethyl-piperidyl-(4)-amino]-cyclobutendiylium-2,4-diolat und dessen Salze mit einer äquivalenten Menge Dodecan-1,12-disäure und Stearinsäure,
1,3-Bis-[N-n-butyl-N-(2,2,6,6-tetramethyl-piperidyl-(4))-amino]-cyclobuten-diylium-2,4-diolat,
1,3-Bis-[N-isobutyl-N-(2,2,6,6-tetramethyl-piperidyl-(4))-amino]-cyclobuten-diylium-2,4-diolat,
1,3-Bis-[N-n-hexyl-N-(2,2,6,6-tetramethyl-piperidyl-(4))-amino]-cyclobuten-diylium-2,4-diolat,
1,3-Bis-[2,2,6,6-tetramethyl-piperidyl-(4)-oxypropylamino]-cyclobuten-diylium-2,4-diolat.

Die Stabilisierungsmittel werden in Mengen von 0,01 bis 2, insbesondere in Mengen von 0,1 bis 0,5 Gewichtsprozent, bezogen auf die Kunststoffe, verwendet.

Geeignete Kunststoffe sind Homo- und Copolymerisate von Olefinen oder Diolefinen, Polyisopren, Polybuten, Polypropylen, Polyethylen niederer und hoher Dichte, Polybutadien, gesättigte und ungesättigte Ethylen-Propylen-Copolymerisate, Ethylen-Buten-Copolymerisate, Ethylen-Vinylacetat-Copolymerisate, Butadien-Styrol-Copolymerisate, Butadien-Styrol-Acrylnitril-Copolymerisate, ferner Homo- und Copolymerisate des Styrols und seiner Homologen, wie z. B. Polystyrol, Styrol-Butadien-Copolymerisate, ferner sogenannte schlagfeste Polystyroltypen, wie Pfropfpolymerisate des Styrols mit Elastomeren oder auch Mischungen von Homo-, Co- oder Pfropfpolymerisaten, außerdem Homo-, Co- und Pfropfpolymerisate des Vinylchlorids, wie Polyvinylchlorid, Polyvinylidenchlorid, Vinylchlorid-Vinylidenchlorid-Copolymerisate, Vinylchlorid-Vinylacetat-Copolymerisate, oder auch Polyvinylacetat oder gesättigte und ungesättigte Polyester, wie Polyethylenterephthalat, Polybutylenterephthalat, Polyetherester, Polyurethane, Polyisocyanurate, Polyacrylnitril und deren Copolymerisate oder Polykondensate, z.B. auf Basis Acetophenon und Formaldehyd.

Besonders geeignet sind Polyamide, z. B. Homopolyamide, wie sie in üblicher Weise aus Lactamen mit mehr als 5 Kohlenstoffatomen im Ring, wie Caprolactam, Capryllactam, Laurinlactam, Undecyllactam, Oenanthlactam, bzw. den entsprechenden ω-Aminosäuren oder ω,ω'-Diaminen und ω,ω'-Dicarbonsäuren mit mehr als 3 Kohlenstoffatomen zwischen den funktionellen Gruppen hergestellt werden können, ferner die entsprechenden Copolyamide. Beispiele für derartige ω,ω'-Diamine sind Tetramethylendiamin, Hexamethylendiamin, Trimethylhexamethylendiamin, Isophorondiamin, Octamethylendiamin und Dodecamethylendiamin. Geeignete Säuren sind z. B. Adipinsäure, Pimelinsäure, Korksäure, Acelainsäure, Sebacinsäure, Undecandicarbonsäure, Dodecan-1,12-dicarbonsäure, Terephthalsäure und Isophthalsäure.

Vorzugsweise werden die Homo- und Copolyamide des Laurinlactams verwendet.

Vorteilhaft ist auch der Einsatz in Polyestern, Polyetherestern, Polyetheresteramiden und Polyurethanen.

Die Kunststoffe können neben den erfindungsgemäßen Verbindungen weitere Zusätze, wie Pigmente, Farbstoffe, Weichmacher oder auch — falls gewünscht — zusätzliche Stabilisatoren oder gegebenenfalls auch Treibmittel enthalten.

Die Stabilisierungsmittel gemäß der Erfindung lassen sich auf bekannte Weise den Kunststoffen zufügen. Zum Beispiel können die Bisamide vor oder während der Polymerisation bzw. Polykondensation den Monomeren zugegeben werden oder sie können in Knetern oder Strangpressen in die Formmassen eingearbeitet werden. Sie können aber auch in Lösungen der Kunststoffe zugesetzt werden, aus denen nach Entfernen des Lösungsmittels z. B. Pulver für Überzugsmittel oder Folien hergestellt werden. Die Art der Einarbeitung richtet sich hier in üblicher Weise nach der Art des Kunststoffes, seiner Herstellung oder seiner Verarbeitung. Außerdem können sie bei der Herstellung der Formkörper in die Formmassen eingebracht oder — falls besonders gewünscht — auf die Formkörper in geeigneter Weise, z. B. durch Auftrommeln oder Aufsprühen in Form einer Lösung, aufgebracht werden. So ist es möglich, die Stabilisierungsmittel für Kunststoffe einzusetzen, die zur Herstellung von Fasern, Folien, Platten oder anderen extrudierten oder spritzgegossenen Formkörpern dienen. Die geformten oder ungeformten Kunststoffe können auch in Form von Latices oder zur Herstellung von Schaumstoffen eingesetzt werden.

Ein Anwendungsschwerpunkt liegt in der Herstellung von Formteilen, die auch bei langandauernder

Belichtung, vor allem im Freien, die guten mechanischen Eigenschaften und damit verbunden ihr vorteilhaftes Aussehen nicht einbüßen dürfen.

In den folgenden Beispielen und Tabellen werden Herstellung, physikalische Eigenschaften und Verwendung einiger Vertreter der Bisamide näher beschrieben.

Beispiel 1

1,3-Bis-[2,2,6,6-tetramethyl-piperidyl-(4)-amino]-cyclobutendiylium-2,4-diolat.

114,0 g Quadratsäudre (1,0 Mol), 390,8 g (2,5 Mol) 4-Amino-2,2,6,6-tetramethyl-piperidin und 800 $cm^3$ Isopropanol wurden im Autoklav fünf Stunden auf 140 °C und anschließend 15 Stunden auf 210 °C erhitzt. Nach dem Abkühlen wurde der Feststoff abgesaugt und getrocknet. Das erhaltene Rohprodukt wurde 3 mal mit siedendem Methanol eluiert, mit kaltem Methanol nachgewaschen und dann scharf getrocknet. Es verblieben 353,1 g weiße Kristalle ; Ausbeute 90,4 % ; Smp. > 400 °C (Z).

| | | | | | |
|---|---|---|---|---|---|
| $C_{22}H_{38}N_4O_2$ (390,57) : | ber. | C 67,65 | H 9,81 | N 14,34 | O 8,19 |
| | gef. | C 67,67 | H 9,79 | N 14,35 | O 8,35 %. |

Beispiel 1.1

Laurinsäuresalz.

Eine Mischung aus 390,6 g (1 Mol) 1,3-Bis-[2,2,6,6-tetramethylpiperidyl-(4)-amino]-cyclobuten-diylium-2,4-diolat und 400 g (2 Mol) Laurinsäure wurde solange auf 200 °C erhitzt, bis das IR-Spektrum das Produkt als Quadratsäuresalz auswies.

Beispiel 1.2

Stearinsäuresalz.

Die Herstellung erfolgte analog zu Beispiel 1.1.

Beispiel 1.3

Benzoesäuresalz.

Die Herstellung erfolgte analog zu Beispiel 1.1.

Beispiel 1.4

Abietinsäuresalz.

Die Herstellung erfolgte analog zu Beispiel 1.1.

Beispiel 1.5

Dodecan-1,12-disäure.

Die Herstellung erfolgte analog zu Beispiel 1.1.

Beispiel 2

1,3-Bis-[N-n-propyl-N-(2,2,6,6-tetramethyl-piperidyl-(4))-amino]-cyclobuten-diylium-2,4-diolat.

In einem 1 l-Rührkolben mit aufgesetztem Wasserauskreiser wurden unter Rühren 363,0 g (1,83 Mol) 4-n-Propylamino-2,2,6,6-tetramethyl-piperidin, 10,8 g (0,095 Mol) Quadratsäure und 150 $cm^3$ Ethylhexanol fünf Stunden unter Rückfluß zum Sieden erhitzt, wobei die Sumpftemperatur 205 °C betrug und 3,0 $cm^3$ Wasser ausgekreist wurden. Nach dem Einengen der Reaktionslösung fielen 33,9 g Kristalle aus ; Ausbeute : 75,2 %, Smp. 198 bis 199 °C (aus Cyclohexan), Extinktion (g/l · cm) bei 278 nm : 27, bei 335 nm : 95.

| | | | | | |
|---|---|---|---|---|---|
| $C_{28}H_{50}O_2N_4$ (474,70) : | ber. | C 70,84 | H 10,62 | N 11,80 | O 6,74 |
| | gef. | C 70,82 | H 10,63 | N 11,68 | O 6,92 %. |

Beispiel 3

1,3-Bis-[N-n-butyl-N-(2,2,6,6-tetramethyl-piperidyl-(4))-amino]-cyclobuten-diylium-2,4-diolat.

In einem 1 l-Rührkolben mit aufgesetztem Wasserauskreiser wurden unter Rühren 392,9 g (1,85 Mol) 4-n-Butylamino-2,2,6,6-tetramethylpiperidin, 10,0 g (0,0877 Mol) Quadratsäure und 150 $cm^3$ 2-Ethylhexanol unter Rückfluß zum Sieden erhitzt, wobei die Sumpftemperatur von 205 auf 217 °C stieg und 2,8 $cm^2$ Wasser ausgekreist wurden.

Dann wurde die Reaktionslösung destilliert. Der Rückstand wurde mit Petrolether aufgekocht. Nach dem Abkühlen, Abnutschen und Trocknen wurden 38,9 g erhalten (88,2 % Ausbeute) ; Smp. : 151 bis 152 °C, Extinktion (g/l · cm) bei 278 nm : 25, bei 335 nm : 83.

$C_{30}H_{54}N_4O_2$ (502,79) : Ber. C 71,67 H 10,83 N 11,14 O 6,36
gef. C 71,35 H 10,88 N 11,01 O 6,56 %.

Beispiel 4

1,3-Bis-[N-isobutyl-N-(2,2,6,6-tetramethyl-piperidyl-(4))-amino]-cyclobuten-diylium-2,4-diolat.

In einem 2 l-Rührkolben mit aufgesetztem Wasserauskreiser wurden unter Rühren 998,2 g (4,70 Mol) 4-Isobutylamino-2,2,6,6-tetramethylpiperidin, 28,0 g (0,246 Mol) Quadratsäure und 400 $cm^3$ Ethylhexanol 12 Stunden unter Rückfluß zum Sieden erhitzt, wobei die Sumpftemperatur nach sieben Stunden den Endwert von 220 °C erreichte und 8,0 $cm^3$ Wasser ausgekreist wurden. Aus der Reaktionslösung fielen 102,1 g Kristalle aus. Aus dem Destillationsrückstand des Filtrats wurden weitere 13,0 g gewonnen. Ausbeute : 93,1 % ; Smp. : 230 bis 232 °C (aus Methylcycohexan), Extinktion (g/l · cm) bei 278 nm : 22, bei 335 nm : 80.

$C_{30}H_{54}N_4O_2$ (502,79) : ber. C 72,10 H 10,98 N 10,71 O 6,53
gef. C 71,67 H 10,83 N 11,14 O 6,36 %.

Beispiel 5

1,3-Bis-[N-n-hexyl-(2,2,6,6-tetramethyl-piperidyl-(4))-amino]-cyclobuten-diylium-2,4-diolat.

In einem 2 l-Rührkolben mit aufgesetztem Wasserauskreiser wurden unter Rühren 1 155,2 g (4,75 Mol) 4-n-Hexylamino-2,2,6,6-tetramethyl-piperidin, 27,4 g (0,24 Mol) Quadratsäure und 250 $cm^3$ Xylol 2,5 Stunden unter Rückfluß zum Sieden erhitzt, wobei der Feststoff noch vor Erreichen des Rückflusses oberhalb von 150 °C in Lösung ging. Die Sumpftemperatur betrug 202 °C. Die ausgekreiste Wassermenge betrug 8,0 $cm^3$. Es wurden 113,7 g erhalten ; Ausbeute 84,8 % d. Th. ; Smp. : 152 bis 153 °C (aus Methylcyclohexan), Extinktion (g/l · cm) bei 328 nm : 76.

$C_{34}H_{62}O_2N_4$ (558,90) : ber. C 73,07 H 11,18 N 10,02 O 5,73
gef. C 73,26 H 11,18 N 10,00 O 5,27 %.

Beispiel 6

1,3-Bis-[N-n-octyl-N-(2,2,6,6-tetramethyl-piperidyl-(4))-amino]-cyclobuten-diylium-2,4-diolat.

In einem 1 l-Rührkolben mit aufgesetztem Wasserauskreiser wurden unter Rühren 339,6 g (1,26 Mol) 99,4 %iges 4-n-Octylamino-2,2,6,6-tetramethyl-piperidin, 7,2 g (0,063 Mol) Quadratsäure und 150 $cm^3$ Ethylhexanol 2,5 Stunden unter Rückfluß zum Sieden erhitzt, wobei der Feststoff noch vor Erreichen des Rückflusses oberhalb von 150 °C in Lösung ging. Die Sumpftemperatur betrug 220 °C. Die ausgekreiste Wassermenge betrug 2,2 $cm^3$. Aus der Reaktionslösung fielen Kristalle aus. Nach dem Waschen mit Petrolether und Trocknen wurden 25,8 g erhalten ; Ausbeute 84,4 % d. Th. ; Smp. : 171 bis 175 °C (aus Cyclohexan), Extinktion (g/l · cm) bei 278 nm : 21, bei 335 nm : 77.

$C_{38}H_{70}N_4O_2$ (615,01) : ber. C 74,21 H 11,47 N 9,11 O 5,20
gef. C 74,30 H 11,58 N 8,98 O 4,96 %.

Beispiel 7

1,3-Bis-[N-2-hydroxyethyl-N-(2,2,6,6-tetramethyl-piperidyl-(4))-amino]-cyclobuten-diylium-2,4-diolat.

5,7 g Quadratsäure (0,05 Mol) und 50,1 g (0,244 Mol) 97,7 %iges 4-(2-Hydroxyethyl)-amino-2,2,6,6-tetramethyl-piperidin wurden fünf Stunden auf 200 °C erhitzt. Nach dem Abkühlen wurde das Reaktionsprodukt mit 150 $cm^3$ Aceton aufgekocht und nach abermaligem Abkühlen abgesaugt und mit Aceton nachgewaschen. Es wurden 16,0 g erhalten ; Ausbeute : 66,8 % d. Th. ; Smp. : 309 bis 311 °C, Extinktion (g/l · cm) bei 336 : 97.

$C_{26}H_{46}N_4O_4$ (478,68) : ber. C 65,24 H 9,69 C 11,71 O 13,37
gef. C 65,05 H 9,72 C 11,70 O 13,46 %.

Beispiel 8

1,3-Bis-[1,2,2,6,6-pentamethyl-piperidyl-(4)-amino]-cyclobutendiylium-2,4-diolat.

58,6 g (0,15 Mol) 1,3-Bis-[2,2,6,6-tetramethyl-piperidyl-(4)-amino]-cyclobuten-diylium-2,4-diolat, 177,2 g Formalin (1,77 Mol $CH_2O$) und 55,8 g (1,2 Mol) 94 %ige HCOOH wurden erhitzt, wobei der Feststoff in Lösung ging. Nachdem die Lösung sieben Stunden unter Rückfluß zum Sieden erhitzt worden war, wurde abgekühlt und die saure Lösung mit Natronlauge auf pH 9 gebracht, wobei ein Niederschlag ausfiel. Dieser wurde mit $H_2O$ nachgewaschen : 40,2 g (64,0 % Ausbeute), weiße Kristalle ; Smp. : > 340 °C (aus Cyclohexanol), Extinktion (g/l · cm) (in DMF) bei 324 nm : 64.

$C_{24}H_{42}N_4O_2$ (418,62) : ber. C 68,86 H 10,11 N 13,38 O 7,64
gef. C 68,75 H 10,25 N 13,26 O 7,75 %.

Beispiel 9

1,3-Bis-[2,2,6,6-tetramethyl-piperidyl-(4))-oxypropylamino]-cyclobuten-diylium-2,4-diolat.

Die Aufschlämmung von 36,5 g (0,32 Mol) Quadratsäure in 510,0 g (2,21 Mol) 94,9 %igem 4-Aminopropyloxy-2,2,6,6-tetramethyl-piperidin wurde zwei Stunden auf 160 °C und dann 1,5 Stunden auf 180 °C erhitzt, wobei 5,3 $cm^3$ Wasser ausgekreist wurden und die Festkörper in Lösung gingen. Beim Abkühlen fiel das Rohprodukt aus, das abgenutscht, zweimal mit Hexan gewaschen und dann getrocknet wurde. Es wurden 154,4 g Kristalle erhalten ; Ausbeute : 95,2 % d. Th. ; Smp. : 218 bis 219 °C (aus Isopropanol), Extinktion (g/l · cm) bei 267 nm : 31, bei 310 nm : 71.

$C_{28}H_{50}O_4N_4$ (506.74) : ber. C 66,37 H 9,95 N 11,06 O 12,63
gef. C 66,05 H 9,91 N 10,96 O 12,83 %.

Beispiel 10

Mit Quadratsäurebisamiden behandelte Kunststoffe

a) Polyamid 12

Die Quadratsäurebisamide wurden auf Polyamid 12-Granulaten aufgetrommelt und das erhaltene Gemisch in einem Zweischneckenextruder homogenisiert. Die auf diese Weise erhaltenen Granulate wurden sodann zu 1 mm starken Platten gepreßt, aus denen 30 × 10 mm große Plättchen geschnitten wurden.

b) Polyetherester

In eine Polyetheresterschmelze auf der Basis Terephthalsäure, 1,4-Butandiol und Polytetrahydrofuran wurden 0,5 Gewichtsprozent des jeweiligen Quadratsäureamids eingetragen. Nach Aufarbeitung zu Granulaten wurden aus diesen 1 mm starke Preßplatten hergestellt, die zu 30 × 10 mm Plättchen verschnitten wurden.

c) Polypropylen (mit einem J-Wert von 330 und einem Molekulargewicht von 380 000)

Der Stabilisator wurde mit dem Polypropylenpulver intensiv vermischt. Die pulvrige Mischung wurde im Schneckenextruder homogenisiert. Die erhaltenen Granulate wurden zu Platten gepreßt.

d) Polystyrol (mit einem Molekulargewicht von 260 000)

Die Herstellung erfolgte analog wie beim Polyamid 12.

e) Acetophenon-Formaldehyd-Harz

Eine Mischung aus 14 Teilen eines Harzes auf der Basis Acetophenon und Formaldehyd, 3 Teilen Weichmacher (Adipin- und Phthalsäureester), 83 Teilen Füllstoffen (Titandioxid und Kreide) und 0,2 Teilen Stabilisator wurde bis zur Schmelze erwärmt und anschließend Platten mit einer Stärke von 3 mm gegossen.

Vergleichsbeispiel A

TINUVIN® 770

Es handelt sich um den Sebacinsäuredi-4-hydroxy-2,2,6,6-tetramethyl-piperidinester, der unter dem Namen TINUVIN® 770 als Warenzeichen der Fa. Ciba-Geigy, Basel, eingetragen ist. Der Ester ist nach der DE-OS 19 29 921 zugänglich.

Vergleichsbeispiel B

HOSTAVIN®

Es handelt sich um 2,2,4,4-Tetramethyl-3,20-diaza-7-oxa-21-oxodispiro (5,1,11,2)heneicosan, das unter dem Namen HOSTAVIN® als Warenzeichen der Fa. Hoechst AG, Frankfurt, eingetragen ist. Die Substanz ist nach der DE-OS 2 606 026 zugänglich.

Vergleichsbeispiel C

1,3-Bis-(diethylamino)-cyclobuten-diylium-2,4-diolat

Die Darstellung erfolgte nach der DE-OS 2 638 855.

Vergleichsbeispiel D

1,3-Bis-(dibutylamino)-cyclobuten-diylium-2,4-diolat

Die Darstellung erfolgte nach der DE-OS 2 638 855.

Vergleichsbeispiel E

1,3-Bis-(cis-2,6-dimethylmorpholino)-cyclobuten-diylium-2,4-diolat

Die Darstellung erfolgte nach der DE-OS 2 638 855.

Vergleichsbeispiel F

1,3-Bis-[(2-hydroxy-1-methylethyl)-amino]-cyclobuten-diylium-2,4-diolat
Die Darstellung erfolgte nach der DE-OS 2 638 855.

Vergleichsbeispiel G

1,3-Bis-piperidino-cyclobuten-diylium-2,4-diolat
Die Darstellung erfolgte nach der DE-OS 2 638 855.

Vergleichsbeispiel H

TINUVIN® 120
Es handelt sich um einen substituierten Benzoesäurephenylester der Fa. Ciba-Geigy, Basel.

Vergleichsbeispiel I

TINUVIN® 622
Es handelt sich um einen Oligoester aus Bernsteinsäure und 1-Hydroxyethyl-4-hydroxy-2,2,6,6-tetramethylpiperidin der Fa. Ciba-Geigy, Basel.

Zur Prüfung der Flüchtigkeit wurden die Quadratsäurebisamide bzw. die mit diesen behandelten Kunststoffe einer thermogravimetrischen Bestimmung unterworfen (vgl. Tabellen 1 und 6). Die Aufheizgeschwindigkeit betrug 10 °C/min.

Die Löslichkeitswerte in Wasser und verdünnter Essigsäure (Tabellen 2 und 3) sowie die Extraktionswerte (Tabellen 4 und 7) belegen die geringe Migrationsneigung der erfindungsgemäßen Verbindungen.

Der Belichtungstest erfolgte mit einer Apparatur, die mit Ultravitaluxlampen ausgerüstet war. Dabei wurde die Zeit gemessen, bis zu der die in Richtung der unbestrahlten Seite und 180° gebogenen Plättchen gebrochen waren (« Knicktest », Tabellen 5 und 9). Proben bestrahlter Plättchen wurden in einer Mischung aus 60 % Phenol und 40 % 1,1,2,2-Tetrachlorethan aufgelöst und die konzentrationsbezogene Viskositätsänderung (J-Wert) nach DIN 53 728 zeitlich verfolgt (Tabelle 8).

Schließlich wurden die Plättchen nach Bestrahlung einer optischen Untersuchung auf Vergilbung unterzogen (Tabelle 10 und 11).

Tabelle 1

Flüchtigkeit der reinen Substanz

| Stabilisator, beschrieben in Beispiel | Gewichtsabnahme von 2 % | 50 % | 80 % |
|---|---|---|---|
| | bei einer Temperatur (°C) von: | | |
| 1 | 350 | 400 | 420 |
| 8 | 350 | 410 | 425 |
| 9 | 310 | 350 | 400 |
| A | 220 | 285 | 300 |
| B | 200 | 280 | 300 |
| C | 150 | 210 | 225 |
| D | 155 | 215 | 230 |
| E | 210 | 260 | 280 |

(Siehe Tabelle 2 Seite 8 f.)

Tabelle 2

Löslichkeit der reinen Substanz in Wasser

| Stabilisator, beschrieben in Beispiel | Löslichkeit (g/100 g $H_2O$) bei 22 °C | 100 °C |
|---|---|---|
| 1 | < 0,002 | 0,015 |
| 3 | < 0,01 | 0,08 |
| 4 | < 0,01 | 0,07 |
| 5 | < 0,01 | 0,03 |
| 6 | < 0,01 | 0,02 |
| 9 | 0,24 | 0,95 |
| C | 240,2 | nicht bestimmt |

Tabelle 3

Löslichkeit der reinen Substanz in 10 %iger Essigsäure bei 22 °C

| Stabilisator, beschrieben in Beispiel | Löslichkeit (g/100 g 10 %ige HAc) |
|---|---|
| 1.1 | 4,6 |
| 1.2 | 2,4 |
| 1.3 | 5,0 |
| 1.4 | < 0,1 |
| 1.5 | 0,9 |
| A | 40,2 |
| F | 22,2 |

Tabelle 4

Extraktion von Stabilisatoren aus Polyamid 12 mit siedenden, organischen Lösemitteln (vgl. Beispiel 10a)

| Stabilisator, beschrieben in Beispiel | Konzentration des Stabilisators im Polyamid 12 (Gew.-%) | Extraktionsgrad nach 8-stündiger Behandlung (% der max. extrahierbaren Menge) in Methanol | Chloroform |
|---|---|---|---|
| 1 | 0,25 | 22,2 | 9,6 |
| A | 0,25 | 68,7 | 69,9 |
| B | 0,25 | 83,1 | 97,2 |

(Siehe Tabelle 5 Seite 9 f.)

Tabelle 5

Belichtungstest mit Stabilisatoren in Polyamid 12 (vgl. Beispiel 10a)

| Stabilisator, beschrieben in Beispiel | Konzentration des Stabilisators im Polyamid 12 (Gew.-%) | Stabilisierungswirkung in Polyamid 12, Belichtung (h), bis Bruch | |
|---|---|---|---|
| | | ohne Costabilisator | mit Costabilisator*) |
| 1 | 0,25 | 790 | 1 076 |
| 1.2 | 0,25**) | 780 | 1 120 |
| 1.3 | 0,25**) | 683 | 976 |
| 1.5 | 0,25**) | 720 | 1 121 |
| 3 | 0,25 | 730 | 1 035 |
| 4 | 0,25 | 840 | 1 120 |
| 5 | 0,25 | 805 | 1 090 |
| 9 | 0,25 | 820 | 1 135 |
| C | 0,25 | 460 | 511 |
| E | 0,25 | 432 | 473 |
| G | 0,25 | 530 | 619 |

*) Als Costabilisator wurde eine Mischung eingesetzt, die — bezogen auf das Polyamid 12 — folgende Komponenten enthielt:

0,25 Gewichtsprozent 2-(3,5-Di-tert.-butyl-2-hydroxyphenyl)-benztriazol
0,25 Gewichtsprozent N,N'-Bis-[3-(3,5-di-tert.-butyl-4-hydroxyphenyl)-propionyl]-hexamethylendiamin
0,25 Gewichtsprozent Tris-(2,4-di-tert.-butylphenyl)-phosphit

**) Die Angabe der Konzentration bezieht sich auf das dem Salz zugrunde liegende Quadratsäurebisamid.

Tabelle 6

Flüchtigkeit der Stabilisatoren aus Polyetherestern*) (vgl. Beispiel 10b)

| Stabilisator, beschrieben in Beispiel | Sublimationsgrad nach 24stündiger Behandlung bei 195 °C und 0,1 mbar (% der max. sublimierbaren Menge) |
|---|---|
| 1 | 13 |
| 7 | 4 |
| A | 88 |

*) Es handelt sich hier wie auch in den Tabellen 7 und 8 um ein Polymer auf der Basis Terephthalsäure, 1,4-Butandiol und Polytetrahydrofuran mit $\overline{M}_n$ = 1 000.

Tabelle 7

Extraktion der Stabilisatoren aus Polyetherestern mit siedenden, organischen Lösemitteln (vgl. Beispiel 10b)

| Stabilisator, beschrieben in Beispiel | Extraktionsgrad nach 8stündiger Behandlung (% der max. extrahierbaren Menge) in | |
|---|---|---|
| | Methanol (65 °C) | Toluol (110 °C) |
| 1 | 12 | 5 |
| 7 | 33 | 25 |
| A | 60 | 53 |

Tabelle 8

Belichtungstest mit Stabilisatoren in Polyetherestern (vgl. Beispiel 10b)

| Stabilisator, beschrieben in Beispiel | Konzentration des Stabilisators im Polyetherester (Gew.-%) | Abnahme des J-Wertes in % nach 20 Tagen | 40 Tagen |
|---|---|---|---|
| 1 | 0,5 | 3 | 5 |
| 7 | 0,5 | 1 | 4 |
| A | 0,5 | 5 | 8 |
| G | 0,5 | 15 | 22 |

Tabelle 9

Belichtungstest mit Stabilisatoren in Polypropylen (vgl. Beispiel 10c)

| Stabilisator, beschrieben in Beispiel | Konzentration des Stabilisators im Polypropylen (Gew.-%) | Stabilisierungswirkung in Polypropylen, Belichtung (h) bis Bruch |
|---|---|---|
| 7 | 0,2 | 375 |
| A | 0,2 | 277 |
| G | 0,2 | 212 |

Tabelle 10

Stabilisierungswirkung bei Polystyrol (vgl. Beispiel 10d)

| Stabilisator, beschrieben in Beispiel | Konzentration des Stabilisators im Polystyrol (Gew.-%) | Yellowness-Index* nach einer Bestrahlungszeit von 750 Std. | 1 230 Std. |
|---|---|---|---|
| 4 | 0,11 | 4,0 | 7,0 |
| 4 | 0,2 | 3,4 | 5,7 |
| 9 | 0,11 | 4,5 | 7,5 |
| E | 0,11 | 10,2 | 15,3 |
| H | 0,11 | 12,9 | 18,9 |

Das eingesetzte Polystyrol enthielt daneben noch 0,056 Gewichtsprozent 2-(2-Hydroxy-5-methyl)-phenyl-2H-benztriazol und 3,5 % Paraffinöl.

* Diese Messung wurde mit einem Suntestgerät entsprechend der ASTMD 1 925-70 durchgeführt. Werte von 0 bis 7,5 entsprechen einem sehr geringen, Werte von 10 bis 20 einem mittelstarken und Werte über 20 einem starken Vergilbungsgrad. Zum Vergleich : Polystyrol, das keinen Stabilisator enthält, weist nach 1 230 Stunden Bestrahlung einen Yellowness-Index von 22,1 auf.

Tabelle 11

Stabilisierungswirkung bei einem Acetophenon-Formaldehyd-Compound (vgl. Beispiel 10e)

| Stabilisator, beschrieben in Beispiel | Konzentration des Stabilisators im Compound | Vergilbungsgrad nach einer Bestrahlungszeit von | |
|---|---|---|---|
| | | 84 Std. | 168 Std. |
| 4 | 0,2 | sehr gering | gering |
| 5 | 0,2 | sehr gering | gering |
| G | 0,2 | mittel | stark |
| I | 0,2 | mittel | stark |

**Patentansprüche**

1. Quadratsäureamidderivate der Formeln Ia bis Ic

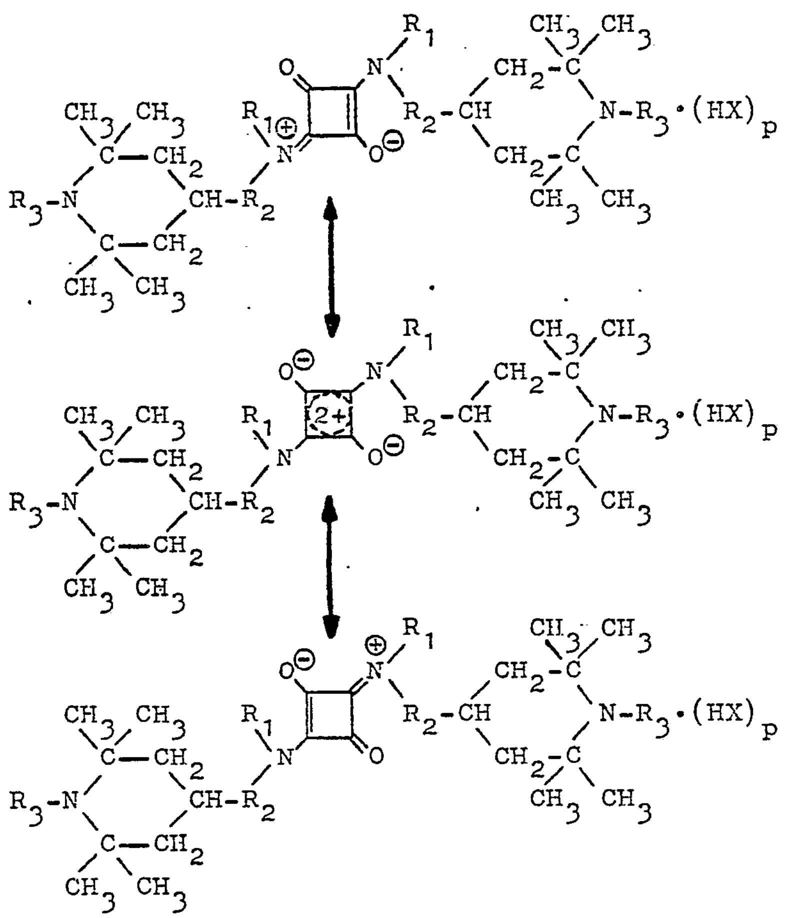

(Ia)

(Ib)

(Ic)

worin $R_2 = -(CH_2)_m-(X)_n-$ mit X = O oder NH ; m = 0 bis 6 ; n = 0 oder 1 ; $0 \leqslant p \leqslant 2$ und $R_1$ Wasserstoff oder ein gegebenenfalls durch eine Hydroxylgruppe substituierter Alkylrest mit 1 bis 12 C-Atomen, $R_3$ Wasserstoff, ein Methyl-, Hydroxyethyl-, Carboxymethyl- oder -ethylrest und HX ein Äquivalent folgender Säuren ist :

a) ein- und mehrbasige organische Säuren mit zu 20 C-Atomen,
b) Schwefel- oder Phosphorsäure und
c) Dodecansulfonsäure oder Phenylphosphonsäure.

2. Quadratsäureamidderivate nach Anspruch 1, worin $R_1$ einen Alkylrest mit 3 bis 8 C-Atomen darstellt, $R_2$ für eine Valenzbindung steht, $R_3$ Wasserstoff ist und p den Wert 0 hat.

3. Quadratsäureamidderivate nach Anspruch 1, worin $R_1$ und $R_3$ Wasserstoff bedeuten und $R_2$ für eine Valenzbindung steht.

4. Verwendung der Quadratsäureamidderivate gemäß Anspruch 1 als Stabilisatoren für Kunststoffe.

5. Verwendung der Quadratsäureamidderivate gemäß Anspruch 1 als Stabilisatoren für Polyester, Polyetherester, Polyetheresteramide oder Polyurethane.

6. Verwendung der Quadratsäureamidderivate gemäß Anspruch 1 als Stabilisatoren für Polyamide.

7. Stabilisierte Kunststoffe, gekennzeichnet durch einen Gehalt von 0,01 bis 2,0 Gewichtsprozent, bezogen auf den unstabilisierten Kunststoff, eines Quadratsäureamidderivats gemäß Anspruch 1.

**Claims**

1. Squaric acid amide derivatives of the formulae Ia to Ic

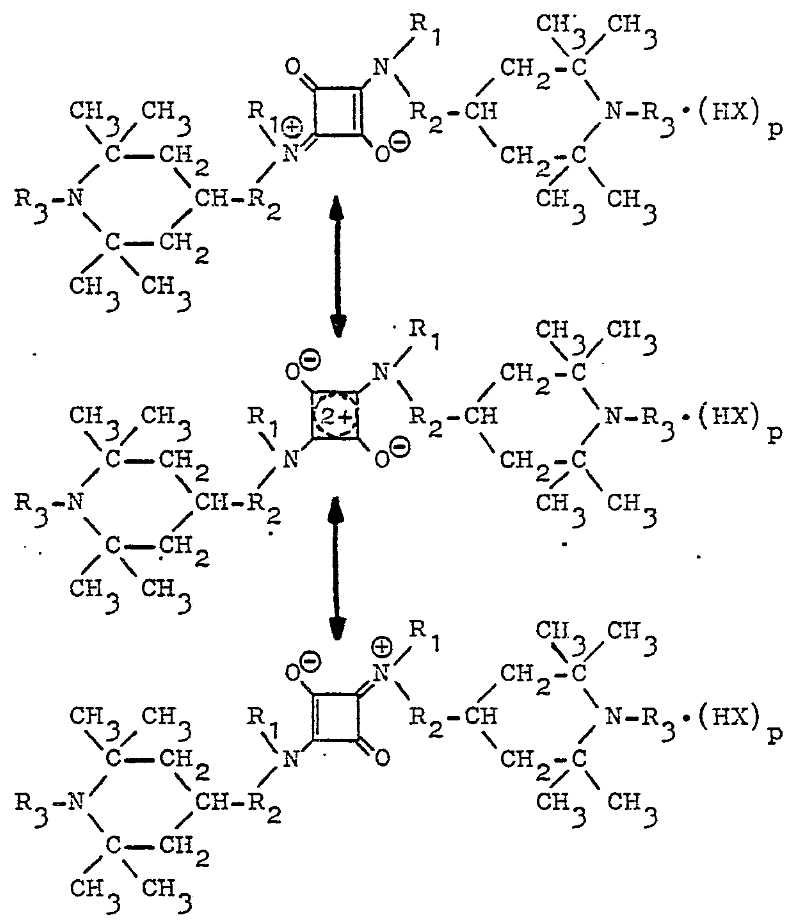

(Ia)

(Ib)

(Ic)

where $R_2$ is $-(CH_2)_m-(X)_n$, X being O or NH, m being zero to 6 and n being zero or 1, p is greater than or equal to zero but less than or equal to 2, $R_1$ is hydrogen or alkyl of 1 to 12 carbon atoms optionally substituted by hydroxyl, $R_3$ is hydrogen, methyl, hydroxyethyl, carbomethyl or carboxyethyl and HX is one equivalent of any of the following acids :

a) monobasic and polybasic organic acids of up to 20 carbon atoms,
b) sulphuric or phosphoric acid, and
c) dodecane-sulphonic acid or phenylphosphonic acid.

2. Squaric acid amide derivatives according to claim 1, where $R_1$ is alkyl of 3 to 8 carbon atoms, $R_2$ is a valence linkage, $R_3$ is hydrogen and p is zero.

3. Squaric acid amide derivatives according to claim 1, where $R_1$ and $R_3$ are hydrogen and $R_2$ is a valence linkage.

4. The use of squaric acid amide derivatives according to claim 1 as stabilisers for plastics.

5. The use of squaric acid amide derivatives according to claim 1 as stabilisers for polyesters, polyetheresters, polyetherestamides or polyurethanes.

6. The use of squaric acid amide derivatives according to claim 1 as stabilisers for polyamides.

7. Stabilised plastics, characterised by a content of 0.01 to 2.0 % by weight, based on the unstabilised plastics material, of a squaric acid amide derivative according to claim 1.

**Revendications**

1. Dérivés amides d'acide quadratique des formules Ia à Ic

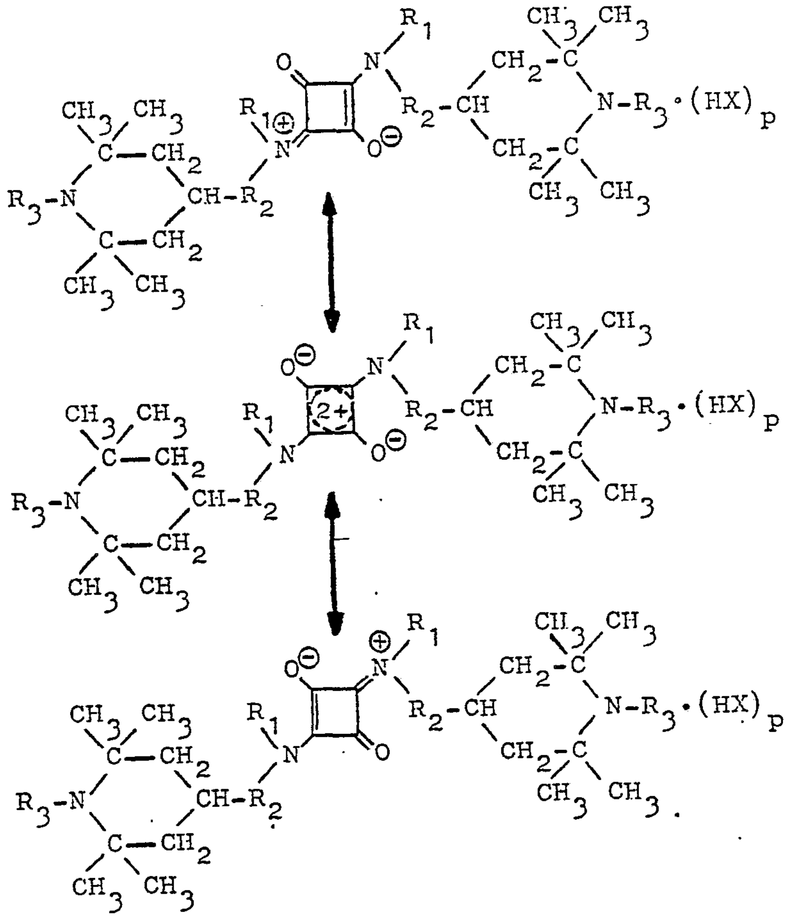

(Ia)

(Ib)

(Ic)

dans lesquelles $R_2 = -(CH_2)_m-(X)_n$ avec X = O ou NH ; m = 0 à 6 ; n = 0 ou 1 ; $0 \leq p < 2$ et $R_1$ est l'hydrogène ou un radical alkyle contenant de 1 à 12 atomes de carbone et éventuellement substitué par un groupe hydroxyle, $R_3$ l'hydrogène, un radical méthyle, hydroxy-éthyle, carboxy-méthyle ou carboxy-éthyle et HX un équivalent des acides suivants :

a) des acides organiques mono-fonctionnels et polyfonctionnels ayant jusqu'à 20 atomes de carbone,

b) l'acide sulfurique ou l'acide phosphorique et

c) l'acide dodécane-sulfonique ou phénylphosphorique.

2. Dérivés amides d'acide quadratique selon la revendication 1, dans lesquels $R_1$ représente un radical alkyle contenant de 3 à 8 atomes de carbone, $R_2$ représente une liaison de valence, $R_3$ est l'hydrogène et p a la valeur zéro.

3. Dérivés amides d'acide quadratique selon la revendication 1, dans lesquels $R_1$ et $R_3$ représentent l'hydrogène et $R_2$ représente une liaison de valence.

4. L'utilisation des dérivés amides d'acide quadratique selon la revendication 1 comme stabilisants pour matières synthétiques.

5. L'utilisation des dérivés amides d'acide quadratique selon la revendication 1 comme stabilisants pour polyesters, polyéthers-esters, polyéthers-esteramides ou polyuréthannes.

6. L'utilisation des dérivés amides d'acide quadratique selon la revendication 1 comme stabilisants pour polyamides.

7. Matières synthétiques stabilisées, caractérisées par une teneur de 0,01 à 2,0 % en poids, relativement à la matière synthétique non stabilisée, d'un dérivé amide d'acide quadratique selon la revendication 1.